(19) Europäisches Patentamt
European Patent Office
Office européen des brevets

(11) Publication number: **0 243 129**
**B1**

(12) **EUROPEAN PATENT SPECIFICATION**

(45) Date of publication of patent specification: **06.06.90**

(51) Int. Cl.[5]: **C 07 C 4/06**, C 07 C 11/02 // B01J29/28

(21) Application number: **87303436.7**

(22) Date of filing: **16.04.87**

(54) **Production of unsaturated hydrocarbons by selective cracking.**

(30) Priority: **24.04.86 GB 8610028**

(43) Date of publication of application:
**28.10.87 Bulletin 87/44**

(45) Publication of the grant of the patent:
**06.06.90 Bulletin 90/23**

(84) Designated Contracting States:
**DE FR GB NL**

(56) References cited:
**EP-A-0 057 049**
**EP-A-0 101 291**
**DE-B-2 158 073**

(73) Proprietor: **The British Petroleum Company p.l.c.**
**Britannic House Moor Lane**
**London EC2Y 9BU (GB)**

(72) Inventor: **Sharma, Bushan Kumar**
**The British Petroleum company p.l.c. Chertsey Road**
**Sunbury-on-Thames Middlesex, TW16 7LN (GB)**

(74) Representative: **Krishnan, Suryanarayana Kalyana et al**
**BP INTERNATIONAL LIMITED Patents & Agreements Division Chertsey Road**
**Sunbury-on-Thames Middlesex TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 243 129 B1

Courier Press, Leamington Spa, England.

## Description

The present invention relates to a process for the production of unsaturated hydrocarbons e.g. olefins from a hydrocarbon mixture containing paraffinic normal and branched hydrocarbons by selective cracking of linear paraffins in the mixture in the presence of tectometallosilicates, especially zeolites of the Theta-1 (the so-called "TON") type.

Zeolites are well known natural and synthetic compositions. Many of them have been demonstrated to have catalytic properties for various types of hydrocarbon conversion and related reactions. Zeolites can be defined as ordered porous crystalline aluminosilicates having a framework structure consisting of a rigid regular three dimensional network of $SiO_4$ and $AlO_4$ tetrahedra in which the tetrahedra are cross-linked by sharing the oxygen atoms and which are sufficiently open to accommodate at least water molecules. Such structures generally contain a regular array of small voids interconnected by channels or pores. The dimensions of the voids and channels can range from those of water to those of quite large molecules. For a given framework structure, the dimensions of the voids and channels are limited to a small number of values, which can vary from structure to structure. Thus these structures are capable of absorbing molecules of certain dimensions while rejecting those of dimensions larger than a critical value which varies with structure type. This has led to zeolites being used as molecular sieves. Zeolites belong to a class of materials that can be termed tectoaluminosilicates which comprise (in addition to zeolites) feldspars and feldspathoids. All oxygen atoms forming the tetrahedra are shared, thus the ratio of total aluminium and silicon atoms to oxygen atoms is 1:2.

Zeolites are best characterised according to framework structure type, i.e. on the topology of the framework, irrespective of composition, distribution of different tetraheral atoms, cell dimensions and symmetry. A code consisting of three capital letters has been adopted for each known structure type following the recommendations by IUPAC on zeolite nomenclature ("Chemical Nomenclature, and Formulations of compositions, of Synthetic and Natural Zeolites", IUPAC yellow booklet, 1978) and a compilation of 38 known zeolite structure types has been published by The Structure Commission of the International Zeolite Association ("Atlas of Zeolite Structure Types", by Meier, W. M. and Olsen, D. H. (1978), distributed by Polycrystal Book Service, Pittsburgh, Pa., USA). In addition to the groups classified by known structure type, there is a further group of crystalline zeolite materials whose X-ray diffraction patterns, sorption, ion-exchange and related properties indicate that they do not have known structure types but appear to have new structure types. These new class of zeolites have unidimensional, non-intersecting channels with ten-membered ring openings up to 6 Å in diameter and are classed as the "TON-type" under the three letter coding of structure type. Theta-1 is an example of such a novel porous crystalline aluminosilicate and is described in our published European patent specification No. 0057049.

There are several prior art publications which indicate that feedstocks such as paraffins, especially $C_3$—$C_8$ paraffins can be dehydrogenated to olefins. Of these GB 1507778, GB 1507549 and GB 1537780 all in the name of the British Petroleum Company p.l.c. refer to the use of gallium oxide on a silica or alumina support as a dehydrogenation catalyst. However, the selectivity of gallium oxide on silica for olefins is only viable at relatively low conversions, the selectivity dropping in favour of aromatics formation and (hydro-) cracking reactions at high conversions. In addition these catalysts suffer from coke deposition and deactivate rapidly.

Another class of catalysts are the types disclosed in our EP—A—0042252 which refers to the use of catalysts containing a gallium compound on a low acid support for the dehydroisomerisation of paraffins, e.g. n-butane to isobutene.

In our patent publications GB 1561590 and EP 0024930, gallium compounds supported on zeolites of the MFI, MEL and FER types, ZSM-12, ZSM-8 and zeolite-beta are said to catalyse such dehydrogenation reactions although the products of the reaction are primarily aromatics combined with some cracked products.

A further publication, EP—A—0036292, describes the use of aluminosilicate zeolites for conversion of paraffins to aromatics and (hydro-) cracked products.

EP—A—57049 describes a novel zeolite designated Theta-1, which may be subjected to exchange or impregnation with a metal, for example copper, silver, zinc, aluminium, gallium, indium, thallium, lead, antimony, bismuth, chromium, molybdenum, tungsten, manganese, iron, cobalt, nickel, ruthenium, rhodium, palladium, iridium, platinum or rhenium. Results of an experiment in which n-butane is passed over the calcined hydrogen form of Theta-1 are given.

Generally when producing olefins from paraffins over MFI-type aluminosilicate catalysts, it is the formation of aromatics that has to be suppressed by modification of the MFI-type aluminosilicate. MFI-aluminosilicates are described in the "Atlas of Zeolite Structure Types", by Meier, W. M. and Olsen, D. H. (1978).

Zeolites having unidimensional, non-intersecting channels with ten-membered ring openings up to 6 Å in diameter, of which Theta-1 type is a specific example, are expected to have similar catalytic properties to MFI-type aluminosilicates because of very similar physicochemical properties. MFI- and Theta-1 type aluminosilicates can be prepared with very similar $SiO_2/Al_2O_3$ ratios and with near identical shape-selective sorption characteristics. For example, they both absorb and desorb xylenes. They both can be prepared from very similar gels (including the same template such as diethanolamine) subjected to identical

digestion parameters, see for example our European Patent Specification No. 0057049. Thus it could be reasonably expected that cracking a mixture of normal and branched paraffins over a catalyst containing an un-modified hydrogen-form of Theta-1 type aluminosilicate should lead to a product having a relatively high proportion of methane and a relatively low alkene to total aliphatic (alkane and alkene) ratio if exposed to conditions under which a catalyst containing an unmodified hydrogen-form of the MFI type aluminosilicate leads to formation of a similar product mixture.

Surprisingly, it has now been found that if a catalyst containing an unmodified hydrogen form of Theta-1 type aluminosilicate or another tectometallosilicate of similar structure which is loaded with a transition metal or metal ion in a specific concentration is used for cracking a mixture of normal and branched paraffins, the process is highly selective in cracking the linear paraffins in the mixture to olefins.

Thus it would appear that although both MFI- and TON-type zeolites have a 10-membered ring structure the behaviour of the intersecting channels in MFI-type is different from that of the non-intersecting channels in the TON type.

Accordingly, the present invention is a process for the production of unsaturated hydrocarbons by cracking a feedstock comprising a mixture of normal and branched chain paraffins in the fluid phase in the presence of a catalyst composition comprising a tectometallosilicate, characterised in that the tectometallosilicate is (a) essentially in its calcined, organic free hydrogen form and has the following composition in terms of the mole ratios of the oxides:

$$(0.9\pm0.1)H_{(4-m)}:XO_2:xSiO_2:yH_2O$$

wherein H is a proton, X is one or more of the metals selected from Al, Ga, Zn, Fe, Cr and B, m is the valency of the metal X in the metal oxide $XO_2$, x is at least 30, y/x is from 0 to 5 and the tectometallosilicate has unidimensional, non-intersecting channels with ten-membered ring openings up to 6 Å in diameter and (b) loaded with a metal or metal ion selected from rhenium, nickel, palladium and platinum in an amount from 0.05 to 2% w/w of the total catalyst composition.

Thus the tectometallosilicates used in the present invention are suitably of the ZSM-23, EU-2 type, ZSM-48 or Theta-1 type and are preferably of the Theta-1-type structure as described in our European Patent Specification No. 0057049 having in its organo-free hydrogen-form an X-ray diffraction pattern substantially as set forth in Table 1 of this specification.

By the term "organic-free hydrogen-form" is meant here and throughout this specification that the tectometallosilicate has been rendered substantially free of organic material introduced during synthesis or subsequent treatments, and the cation is essentially hydrogen. The tectometallosilicates prepared from organic bases contain organics as syntehsised and these can suitably be removed by calcination in air. The tectometallosilicates prepared from ammonia do not contain organics as synthesised. Therefore, in such a case, an organics removal step such as calcination in air is not essential.

The $H_2O$ content "y" of the tectometallosilicate is the water of hydration and will depend, within the ratios set out above, upon the conditions under which it is dried, calcined, subjected to further aqueous treatments or combinations thereof after synthesis. The $H_2O$ content "y" does not include water which may be notionally present when the cation is hydrogen.

The cation in the tectometallosilicate is essentially H though very small amounts of other cations such as ammonium, alkali metal cations, alkaline earth metal cations, organic nitrogen containing cations, aluminium cation, and mixtures thereof which have not been completely removed during calcination, may also be present.

The cations present in the tectometallosilicate may be replaced using conventional ion exchange techniques either wholly or partially by other cations e.g. hydrogen ions or metal cations.

The organic-free hydrogen-form of the tectometallosilicate may be produced by known methods such as exchange with hydrogen ions or with ammonium cations followed by one or more calcinations or a combination of the two followed by one or more calcination stages, if the tectometallosilicate still contained ammonium ions.

The metal X in the metal oxide $XO_2$ is Al, Ga, Zn, Fe, B or Cr. In the tectometallosilicate the molar ratio of silica to metal X is preferably from 30:1 to 200:1.

Where the tectometallosilicates according to the present invention is Theta-1, it has in its organic-free hydrogen form an X-ray diffraction pattern shown in Table 1 below. The specific values in the Table were determined using copper K-alpha radiation and a computer step scan.

The peak heights, I, and their position as a function of 2-theta, where theta is the Bragg angle, were read from the spectrometer output. From this output the relative intensities $100\times I/I_o$, where $I_o$ is the intensity of the strongest peak, and d the interplanar spacing in A, corresponding to the recorded peaks were calculated.

It will be understood by those skilled in the art that the X-ray diffraction pattern of tectometallosilicates may vary in the values of $I/I_o$ and the d-spacing depending for example upon whether the sample being examined is calcined or uncalcined, upon the temperature of calcination, upon the nature of the cation present in the tectometallosilicate, the mole ratio of silica to metal oxide, and the particle size of the tectometallosilicate.

The tectometallosilicate is suitably produced by mixing a source of silica, a source of alumina, a source

of alkali metal(s), water and an organic or inorganic nitrogen containing base until a homogeneous gel is formed and crystallising the gel at a temperature above 70°C.

TABLE 1

| 2-Theta (degrees) | d-Spacing (Angstroms) | Relative intensity $100 \times I/I_o$ |
|---|---|---|
| 8.06±0.2 | 11.25—10.70 | 50 to 100 |
| 10.06±0.2 | 9.01—8.63 | 5 to 30 |
| 12.69±0.2 | 7.09—6.87 | 10 to 30 |
| 16.28±0.2 | 5.51—5.38 | 5 to 15 |
| 19.40±0.2 | 4.62—4.53 | 5 to 15 |
| 20.26±0.2 | 4.43—4.34 | 50 to 100 |
| 24.11±0.2 | 3.72—3.66 | 50 to 100 |
| 24.52±0.2 | 3.66—3.60 | 30 to 90 |
| 25.65±0.2 | 3.50—3.45 | 15 to 45 |

scanned up to 2 theta=32

The tectometallosilicates having unidimensional, non-intersecting channels with ten-membered ring openings up to 6 Å in diameter are loaded with metals or metal ions having known hydrogenation/dehydrogenation activity. Examples of such metals include the transition metals, especially rhenium, nickel, palladium and platinum. Of these nickel or nickel ions are most preferred.

The tectometallosilicate used herein may be loaded with the relevant transition metals or ions by conventional techniques of ion-exchange or impregnation. For instance, the tectometallosilicate may be loaded with nickel by incipient wetness impregnation of the H-form of the zeolite using aqueous solutions of the nickel salts e.g. nickel (II) nitrate. Nickel salts may also be dissolved in non-aqueous media, e.g. alcohols to form non-aqueous solutions, which can then be used for impregnating the H-zeolite.

The amount of transition metals or ions loaded on to the tectometallosilicate is suitably from 0.05 to 1.0%, preferably from 0.08 to 0.8% by weight of the total catalyst composition.

The tectometallosilicates used in the present invention may be bound in a suitable binding material using methods and binding materials well known in the art such as e.g. silica, alumina and aluminium phosphate. Where a transition metal or ion is incorporated in the tectometallosilicate, it may be bound before or after loading with said metal or ion. The catalyst composition may be subjected to further activation treatments e.g. thermochemically by heating for instance in an inert gas e.g. nitrogen, or in an oxidative environment such as dry air, steam or molecular oxygen, or in a reductive environment such as dry hydrogen prior to contact with the paraffin feestock. Such further treatments may be carried out during one or more of the catalyst preparation stages.

It is preferable to carry out the activation treatment in hydrogen. For instance, such treatment with hydrogen may be carried out using a carrier gas which contains 1—100% v/v, preferably from 30—100% v/v of hydrogen. The treatment temperature is suitably from 400—800°C, preferably from 400—700°C. The treatment pressure may vary from 100 to 5000 kPa but is preferably from 100—2000 kPa. The treatment may be carried out over a duration fo 5 minutes to 200 hours, preferably from 1—20 hours.

It will be appreciated by those skilled in the art that the conditions specified for any of the aforementioned treatments will be interdependent. Increasing the severity of one or more of the parameters may allow a reduction in the severity of the other relevant parameters. In the case of steam treatment for instance raising the treatment temperature can be expected to shorten the duration of the treatment.

The mixture of paraffins which may be cracked to the corresponding olefinic hydrocarbons by contact with the catalyst compositions herein defined are suitably mixtures of saturated hydrocarbons up to the waxes preferably mixtures containing $C_2$—$C_{20}$ paraffins e.g. light naphtha. The feed may also contain olefins and aromatic hydrocarbons e.g. a reformate. The feed may be diluted for instance with hydrogen, nitrogen or steam.

The feature of the cracking reaction is that unlike dehydrogenation, the olefins in the product have a lower number of carbon atoms than the paraffin feedstock being cracked. In addition, the process is selective towards cracking linear paraffins.

The cracking reaction is suitably carried out from 400—700°C, preferably from 450—700°C, the precise temperature depending upon other variables such as feedstock and catalyst activity.

The cracking reaction may be carried out at reduced or elevated pressure, suitably from 10—5000 kPa pressure, preferably from 50—1000 kPa pressure.

The liquid hourly space velocity of the feedstock over the catalyst composition is suitably in the range of 0.1—10 LHSV, preferably 0.5—5 LHSV.

The products of the cracking reaction are principally hydrocarbons which are rich in light olefins e.g.

ethylene, propylene and the butylenes. The cracking reaction is efficient and selective in that by-products such as methane and ethane are formed only in very small amounts.

The products from the reaction can be separated according to their properties e.g. melting point, boiling point, polarity etc. or to their molecular dimensions. Methods such as molecular sieving, distillation, selective adsorption can be used to achieve separation.

The light olefins such as the $C_2$—$C_4$ oelfins are valuable petrochemical feedstock for producing polyolefins, alcohols, surfactants and alkylaromatics, and as a feedstock for producing diesel and jet fuels. The product olefins can also be used for producing methyl tert. butyl ether (MTBE) and for producing olefin oligomers which are useful as gasoline blending components.

The process can be combined with an olefin to hydrocarbon liquids process to give a combined paraffins to hydrocarbon liquids process. The process can also be used for fluid catalytic cracking and for catalytic dewaxing.

The present invention is further illustrated with reference to the following Examples.

Examples

A. Catalyst preparation

The zeolites used in the Examples and Comparative Tests described below were prepared according to the recipes for the respective zeolites in the following patents:

| | |
|---|---|
| Theta-1 | EP 0057049 (The British Petroleum Co.). |
| ZSM-23 | USP 4076842 (Mobil). |
| EU-2 | GB 2077709-A (ICI). |
| ZSM-5 | EP 0030811 (The British Petroleum Co.). |

These "as-synthesised" zeolites were then converted into the H-form as follows:—

The "as-synthesised" zeolites were calcined at 600°C in air, cooled to ambient temperature and then treated with 1 mol $dm^{-3}$ aqueous solutions of ammonium nitrate at 90°C for 4 hours. The impregnation with nickel, where stated, was then carried out by incipient wetness techniques. Thus a known quantity of $NH_4$-zeolite was mixed with sufficient distilled water to produce a thick slurry. A quantity of nickel nitrate hexahydrate was dissolved in an amount of water equal to the zeolite pore volume. The nickel solution was mixed well into the zeolite slurry and the solids left to dry at ambient temperature over 16 hours. The nickel-impregnated zeolite was dried at 90°C, cooled and pressed into pellets (8—16 mesh size BSS) and loaded into the catalytic reactor. The activation procedure used for the individual catalysts are given in the Examples and Tests described below:

Feed:

A mixture of essentially paraffinic hydrocarbons which contains linear and branched alkanes e.g. a light naphtha of the following composition was used in the Examples below:—

| Composition | % wt. |
|---|---|
| i-Pentane | 15.0 |
| n-Pentane | 25.9 |
| 2,2-Dimethylbutane | 0.8 |
| 2,3-Dimethylbutane | 1.6 |
| 2-Methylpentane | 19.9 |
| 3-Methylpentane | 17.3 |
| n-Hexane | 17.6 |
| Benzene | 1.9 |

Comparative Test 1 (not according to the invention)

The light naphtha was cracked under the conditions shown and the products identified by Gas-Liquid Chromatographic techniques as shown in Table 2 below:—

Catalyst: H-Theta-1 Si/Al=32 molar activated in hydrogen at 500°C for 1 hour.

Reaction conditions:

| | |
|---|---|
| Temperature (°C) | 490 |
| Pressure (kPa) | 100 |
| $H_2$:Hydrocarbon (molar feed ratio) | 1:1 |
| LHSV ($h^{-1}$) | 1 |
| Feed | light naphtha |

$$\text{Calculated conversion for reactant i} = \frac{\text{moles i fed} - \text{moles i in product} \times 100\%}{\text{moles i fed}}$$

| Reactant | Conversion mol % |
|---|---|
| n-Hexane | 57.3 |
| n-Pentane | 51.5 |
| i-Pentane | 23.3 |
| 2-Methylpentane | 17.0 |
| 3-Methylpentane | 16.6 |
| 2,2-Dimethylbutane | 0 |
| 2,3-Dimethylbutane | 40.8 |

Example 1

Catalyst: 0.09 %wt Ni impregnated on H-Theta-1 Si/Al=32.
Activation: air 500°C 1 hour, nitrogen 500°C 1 hour, hydrogen 500°C 1 hour.

Reaction conditions:—

| | |
|---|---|
| Temperature (°C) | 550 |
| Pressure (kPa) | 100 |
| Feed | light naphtha |
| $N_2$: Hydrocarbon (molar feed ratio) | 0.8:1 |
| LHSV ($h^{-1}$) | 1 |

| Reactant | Conversion mol % |
|---|---|
| n-Hexane | 37.8 |
| n-Pentane | 35.6 |
| i-Pentane | 25.0 |
| 2-Methylpentane | 12.9 |
| 3-Methylpentane | 14.3 |
| 2,2-Dimethylbutane | 1.3 |
| 2,3-Dimethylbutane | 46.5 |

Comparative Test 2 (not according to the invention)

Catalyst: H-ZSM-5, Si/Al=44

Activation in air, nitrogen and hydrogen at 500°C for 1 hour each.

Reaction conditions:

| | |
|---|---|
| Temperature (°C) | 240 |
| Pressure (kPa) | 100 |
| Feed | light naphtha |
| $H_2$: Hydrocarbon (molar feed ratio) | 0.8:1 |
| LHSV ($h^{-1}$) | 1 |

| Reactant | Conversion mol % |
|---|---|
| n-Hexane | 59.9 |
| n-Pentane | 8.8 |
| i-Pentane | 0 |
| 2-Methylpentane | 36.6 |
| 3-Methylpentane | 27.6 |
| 2,2-Dimethylbutane | 8.9 |
| 2,3-Dimethylbutane | 51.0 |

Example 2

This Example illustrates a process using H-EU-2 zeolite which also has unidimensional, non-intersecting channels with 10-membered ring openings and having a diameter up to 6 Å.

Catalyst: 0.08 wt % Ni impregnated onto H-EU-2 Si/Al=143.

Activated in air and nitrogen at 500°C for 1 hour each.

Reaction conditions:

| | |
|---|---|
| Temperature (°C) | 620 |
| Pressure (kPa) | 100 |
| Feed | light naphtha |
| $N_2$: Hydrocarbon (molar feed ratio) | 0.8:1 |
| LHSV ($h^{-1}$) | 1 |

| Reactant | Conversion mol % |
|---|---|
| n-Hexane | 56.6 |
| n-Pentane | 45.0 |
| i-Pentane | 25.2 |
| 2-Methylpentane | 17.1 |
| 3-Methylpentane | 24.4 |
| 2,2-Dimethylbutane | 5.1 |
| 2,3-Dimethylbutane | 49.0 |

Reaction products

The mode of cracking specific to H-zeolites having unidimensional, non-intersecting channels with 10-ring openings gives products containing large quantities of alkenes.

Example 3

Catalyst: 0.07% w/w Ni impregnated onto H-ZSM-23, Si/Al=40.
Activated in air and nitrogen at 500°C for 1 hour each.

Reaction conditions:

| | |
|---|---|
| Temperature (°C) | 580 |
| Pressure (kPa) | 100 |
| Feed | light naphtha |
| $N_2$: Hydrocarbon (molar feed ratio) | 0.8:1 |
| LHSV ($h^{-1}$) | 1 |

| Reactant | Conversion mol % |
|---|---|
| n-Hexane | 81.9 |
| n-Pentane | 60.9 |
| i-Pentane | 29.2 |
| 2-Methylpentane | 29.4 |
| 3-Methylpentane | 29.5 |
| 2,2-Dimethylbutane | 2.5 |
| 2,3-Dimethylbutane | 81.5 |

Comparative Test 3 (not according to the invention)

Catalyst: 0.08 wt % Ni impregnated onto H-ZSM-5, Si/Al=59.
Activation in air and nitrogen at 500°C for 1 hour each.

Reaction conditions:

| | |
|---|---|
| Temperature (°C) | 550 |
| Pressure (kPa) | 100 |
| Feed | light naphtha |
| $H_2$: Hydrocarbon (molar feed ratio) | 0.8:1 |
| LHSV ($h^{-1}$) | 1 |

| Reactant | Conversion mol % |
|---|---|
| n-Hexane | 67.2 |
| n-Pentane | 57.1 |
| i-Pentane | 52.0 |
| 2-Methylpentane | 48.7 |
| 3-Methylpentane | 54.6 |
| 2,2-Dimethylbutane | 3.8 |
| 2,3-Dimethylbutane | 79.6 |

TABLE 2

| Catalyst Example/Test | Theta-1 (Comp. 1) | Ni-Theta-1 1 | Ni-EU-2 2 | Ni-ZSM-23 3 | H-ZSM-5 (Comp. test 2) | Ni-ZSM-5 (Comp. test 3) |
|---|---|---|---|---|---|---|
| Reaction temp. °C | 490 | 550 | 620 | 580 | 240 | 550 |
| Methane mol % in product gas | 4.1 | 4.6 | 8.1 | 4.8 | 0 | 16.6 |
| Molar ratios of alkene/(alkene+ alkane) in product gas | | | | | | |
| C2=/(C2+C2=) | 0.42 | 0.54 | 0.57 | 0.51 | 0.50 | 0.32 |
| C3=/(C3+C3=) | 0.44 | 0.78 | 0.82 | 0.74 | 0.03 | 0.38 |
| C4=/(C4+C4=) | 0.66 | 0.91 | 0.89 | 0.87 | 0.02 | 0.40 |

The above results show that under the same conditions and using the same feed zeolites having intersecting channels with 10-membered ring pore openings produce predominantly alkanes, relatively lower amounts of olefins and the make of methane is high when compared with the process of the present invention where high olefin values are realised with a low methane content in the product.

**Claims**

1. A process for the production of unsaturated hydrocarbons by cracking a feedstock comprising a mixture of normal and branched chain paraffins in the fluid phase in the presence of a catalyst composition comprising a tectometallosilicate, characterised in that the tectometallosilicate is (a) essentially in its calcined, organic free hydrogen form and has the following composition in terms of the mole ratios of the oxides:

$$(0.9\pm0.1)H_{(4-m)}:XO_2:xSiO_2:yH_2O$$

wherein H is a proton, X is one or more of the metals selected from Al, Ga, Zn, Fe, Cr and B, m is the valency of the metal X in the metal oxide $XO_2$, x is at least 30, y/x is from 0 to 5 and the tectometallosilicate has unidimensional, non-intersecting channels with ten-membered ring openings up to 6 Å in diameter and (b) loaded with a metal or metal ion selected from rhenium, nickel, palladium and platinum in an amount from 0.05—2% w/w of the total catalyst composition.

2. A process according to claim 1 wherein the molar ratio of silica to metal X in the tectometallosilicate is from 30:1 to 200:1.

3. A process according to claim 1 or 2 wherein X is Al or Ga and the tectometallosilicate is Theta-1 whose X-ray diffraction pattern in its calcined, organic-free hydrogen form comprises the peaks and intensities substantially as shown in Table 1 below.

TABLE 1

| 2-Theta (degrees) | d-Spacing (Angstroms) | Relative intensity $100\times I/I_o$ |
|---|---|---|
| 8.06±0.2 | 11.25—10.70 | 50 to 100 |
| 10.06±0.2 | 9.01—8.63 | 5 to 30 |
| 12.69±0.2 | 7.09—6.87 | 10 to 30 |
| 16.28±0.2 | 5.51—5.38 | 5 to 15 |
| 19.40±0.2 | 4.62—4.53 | 5 to 15 |
| 20.26±0.2 | 4.43—4.34 | 50 to 100 |
| 24.11±0.2 | 3.72—3.66 | 50 to 100 |
| 24.52±0.2 | 3.66—3.60 | 30 to 90 |
| 25.65±0.2 | 3.50—3.45 | 15 to 45 |

scanned up to 2 theta=32.

4. A process according to anyone of the preceding claims wherein the catalyst composition comprises the tectometallosilicate loaded with the 0.08—0.8% w/w of the metal or metalions based on the total catalyst composition.

5. A process according to any one of the preceding claims wherein the catalyst composition comprises the tectometallosilicate loaded with the metals or metal ions bound in a binding material.

6. A process according to any one of the preceding claims wherein the catalyst composition is activated thermochemically, or, in an oxidative or a reductive environment prior to contact with the paraffin feedstock being cracked.

7. A process according to claim 6 wherein the activation of the catalyst composition is carried out in hydrogen at a temperature from 400—800°C and a pressure of 100—2000 kPa over a duration of 5 minutes to 200 hours.

8. A process according to any one of the preceding claims wherein the paraffin feedstock being cracked comprises mixtures of $C_2$—$C_{20}$ paraffins.

9. A process according to any one of the preceding claims wherein the paraffin feedstock is cracked at a temperature from 400—700°C, a pressure from 10—5000 kPa and an LHSV of 0.1—10.

**Patentansprüche**

1. Verfahren zur Erzeugung ungesättigter Kohlenwasserstoffe durch Cracken eines eine Mischung aus normalen und kettenverzweigten Paraffinen umfassenden Einsatzmaterials in fließfähiger Phase in Gegenwart einer Katalysator-Zusammensetzung, die ein Tectometallosilicat umfaßt, dadurch gekennzeichnet, daß das Tectometallosilicat (a) im wesentlichen in seiner calcinierten Wasserstoff-Form

frei von organischen Stoffen vorliegt und die folgende Zusammensetzung, ausgedrückt als Stoffmengen-Verhältnis ("Mol-Verhältnis") der Oxide, hat:

$$(0{,}9\pm0{,}1)H_{4-m}:XO_2:xSiO_2:yH_2O,$$

worin

H ein Proton ist,

X für ein oder mehrere der Metalle ausgewählt aus Al, Ga, Zn, Fe, Cr und B steht,

m die Wertigkeit des Metalls X in dem Metalloxid $XO_2$ ist,

x wenigstens 30 ist,

y/x 0 bis 5 ist und

das Tectometallosilicat eindimensionale, kreuzungsfreie Kanäle mit zehngliedrigen Ringöffnungen mit einem Durchmesser bis zu 6 Å aufweist und (b) mit einem Metall oder Metall-Ion, das aus Rhenium, Nickel, Palladium und Platin ausgewählt ist, in einer Menge von 0,05 bis 2 Gew./Gew.-% der gesamten Katalysator-Zusammensetzung beladen ist.

2. Verfahren nach Anspruch 1, worin das Stoffmengen-Verhältnis des Siliciumdioxids zu dem Metall X in dem Tectometallosilicat 30:1 bis 200:1 betägt.

3. Verfahren nach Anspruch 1 oder 2, worin X Al oder Ga ist und das Tectometallosilicat Theta-1 ist, dessen Röntgenbeugungsdiagramm in seiner calcinierten Wasserstoff-Form frei von organischen Stoffen im wesentlichen die Peaks und Intensitäten umfaßt, wie sie in der Tabelle 1 hiernach angegeben sind:

TABELLE 1

| 2-Theta (Grad) | d-Abstand (Angström) | Relative Intensität $100\times I/I_o$ |
|---|---|---|
| 8,06±0,2 | 11,25—10,70 | 50 bis 100 |
| 10,06±0,2 | 9,01—8,63 | 5 bis 30 |
| 12,69±0,2 | 7,09—6,87 | 10 bis 30 |
| 16,28±0,2 | 5,51—5,38 | 5 bis 15 |
| 19,40±0,2 | 4,62—4,53 | 5 bis 15 |
| 20,26±0,2 | 4,43—4,34 | 50 bis 100 |
| 24,11±0,2 | 3,72—3,66 | 50 bis 100 |
| 24,52±0,2 | 3,66—3,60 | 30 bis 90 |
| 25,65±0,2 | 3,50—3,45 | 15 bis 45 |

abgetastet bis zu 2 Theta=32.

4. Verfahren nach irgendeinem der vorhergehenden Ansprüche, worin die Katalysator-Zusammensetzung das Tectometallosilicat, beladen mit 0,08 bis 0,8 Gew./Gew.-% des Metalls oder der Metall-Ionen, bezogen auf die gesamte Katalysator-Zusammensetzung, umfaßt.

5. Verfahren nach irgendeinem der vorhergehenden Ansprüche, worin die Katalysator-Zusammensetzung das Tectometallosilicat, beladen mit den Metallen oder den Metall-Ionen, die in einem bindenden Material gebunden sind, umfaßt.

6. Verfahren nach irgendeinem der vorhergehenden Ansprüche, worin die Katalysator-Zusammensetzung thermochemisch oder in einer oxidativen oder reduktiven Umgebung vor dem Kontakt mit dem dem Cracken unterworfenen Paraffin-Einsatzmaterial aktiviert wird.

7. Verfahren nach Anspruch 6, worin die Aktivierung der Katalysator-Zusammensetzung während einer Zeitdauer von 5 min bis 200 h in Wasserstoff bei einer Temperatur von 400°C bis 800°C und einem Druck von 100 bis 2000 kPa durchgeführt wird.

8. Verfahren nach irgendeinem der vorhergehenden Ansprüche, worin das dem Cracken unterworfene Paraffin-Einsatzmaterial Mischungen aus $C_2$—$C_{20}$-Paraffinen umfaßt.

9. Verfahren nach irgendeinem der vorhergehenden Ansprüche, worin das Paraffin-Einsatzmaterial bei einer Temperatur von 400°C bis 700°C, einem Druck von 10 bis 5000 kPa und einem LHSV-Wert (stündliche Flüssigkeits-Raumgeschwindigkeit) von 0,1 bis 10 durchgeführt wird.

**Revendications**

1. Procédé pour la production d'hydrocarbures insaturés par craquage d'une charge d'alimentation, comprenant un mélange de paraffines normales et de paraffines ramifiées, en phase fluide en présence d'une composition de catalyseur comprenant un tectométallosilicate, procédé caractérisé en ce que le

tectométallosilicate est (a) essentiellement sous sa forme hydrogène calcinée, dépourvue de matières organiques et a la composition suivante, en termes des rapports molaires entre les oxydes:

$$(0,9\pm0,1)H_{(4-m)}:XO_2:xSiO_2:yH_2O$$

(où H représente un proton; X représente un ou plusieurs des éléments ou métaux choisis parmi Al, Ga, Zn, Fe, Cr et B; m est la valence de l'élément métal X dans l'oxyde $XO_2$; x vaut au moins 30; le rapport y/x vaut de 0 à 5) et le tectométallosilicate possède des canaux unidimensionnels, qui ne se coupent pas, ayant des ouvertures ou orifices, en forme de cycles décagonaux (à dix éléments) pouvant aller jusqu'à 6 Å de diamètre, et (b) est chargé d'un métal ou d'un ion de métal choisi parmi le rhénium, le nickel, le palladium et le platine, présents en une quantité représentant de 0,05 à 2% en poids/poids de la composition totale du catalyseur.

2. Procédé selon la revendication 1, dans lequel le rapport molaire de la silice à l'élément métallique X présents dans le tectométallosilicate va de 30:1 à 200:1.

3. Procédé selon la revendication 1 ou 2, dans lequel X représente Al ou Ga, et le tectométallosilicate est Thêta-1, dont le spectre de diffraction des rayons X, sous la forme hydrogène du tectométallosilicate calciné et dépourvu de matières organiques comprend les pics et les intensités, essentiellement comme représenté sur le tableau 1 ci-après:

TABLEAU 1

| 2-thêta (degrés) | Distance d (Angströms) | Intensité relative $100\times I/I_o$ |
|---|---|---|
| 8,06±0,2 | 11,25—10,70 | 50 à 100 |
| 10,06±0,2 | 9,01—8,63 | 5 à 30 |
| 12,69±0,2 | 7,09—6,87 | 10 à 30 |
| 16,28±0,2 | 5,51—5,38 | 5 à 15 |
| 19,40±0,2 | 4,62—4,53 | 5 à 15 |
| 20,26±0,2 | 4,43—4,34 | 50 à 100 |
| 24,11±0,2 | 3,72—3,66 | 50 à 100 |
| 24,52±0,2 | 3,66—3,60 | 30 à 90 |
| 25,65±0,2 | 3,50—3,45 | 15 à 45 |

balayage effectué jusqu'à 2 thêta=32.

4. Procédé selon l'une quelconque des revendications précédentes, dans lequel la composition du catalyseur comprend le tectométallosilicate chargé par la proportion de 0,08 à 0,8% en poids/poids du métal ou des ions métal sur la base de la composition totale du catalyseur.

5. Procédé selon l'une quelconque des revendications précédentes, dans lequel la composition du catalyseur comprend le tectométallosilicate chargé par les métaux ou les ions de métaux liés dans une matière liante.

6. Procédé selon l'une quelconque des revendications précédentes, dans lequel la composition du catalyseur est activée par voie thermochimique ou, dans un environnement oxydant ou dans un environnement réducteur, avant mise en contact avec la charge des paraffines d'alimentation soumises à craquage.

7. Procédé selon la revendication 6, dans lequel l'activation de la composition du catalyseur est effectuée dans de l'hydrogène, à une température de 400 à 800°C et sous une pression de 100 à 2000 kPa, sur une durée allant de 5 min à 200 h.

8. Procédé selon l'une quelconque des revendications précédentes, dans lequel la charge des paraffines d'alimentation soumises à craquage comprend des mélanges de paraffines en $C_2$ à $C_{20}$.

9. Procédé selon l'une quelconque des revendications précédentes, dans lequel la charge des paraffines d'alimentation est craquée à une température de 400 à 700°C, sous une pression de 10 à 5000 kPa et à une vitesse spatiale horaire de liquide (VVH) de 0,1 à 10.